# EUROPEAN PATENT SPECIFICATION

(11) **EP 1 428 492 B1**
(45) Date of publication and mention of the grant of the patent: **17.01.2007**
(21) Application number: 03026306.5
(22) Date of filing: 15.11.2003
(51) Int. Cl.: A61K 6/083

(54) **Light curing type paint resin for shade adjustment**
Lichthärtendes Lackharz zur Anpassung der Schattierungen
Peinture de résine photodurcissable pour la mise au point des nuances

(30) Priority: 09.12.2002 JP 2002357022
(43) Date of publication of application: 16.06.2004
(73) Proprietor: Heraeus Kulzer GmbH, 63450 Hanau (DE)
(72) Inventor: Grundler, Andreas, Dr., 42117 Wuppertal (DE); Onodera, Yasuo, 27-0033 Chiba-Pref. (JP); Erdrich, Albert, Dr., 61231 Bad Nauheim (DE); Hohmann, Alfred, 61389 Schmitten (DE)
(74) Representative: Kühn, Hans-Christian

(56) References cited:
- EP-A- 1 243 230
- US-A- 4 150 485
- US-A- 4 411 625
- US-A- 4 512 743

## Description

The invention relates to a photocurable dental coating material, suitable for color correction of dental restorations or artificial teeth.

In order to satisfy the esthetic elements in dental prosthetic treatment, methods of using not only metals but also ceramics and resins for facings can recreate corona color. Among them, the development of resins is remarkable, and some with the capability of having physical strength with sufficient abrasion resistance (chewing resistance, withstanding tooth brushing) have started to appear. To further improve the esthetics of such rigid resin for dental use, dental technicians are hard at work on the production of prosthetic materials by employing various methods.

One such method is staining, in which colored gel state stain material is used to perform the desired shade adjustment and to characterize the facing. In this method, gel state paste will be colored with a brush, so the operability of the stain material must be high. For that reason, the viscosity and degree of polymerization has been regarded as more important than abrasion resistance, and thus either filler would not be filled in, or even if it were, it was limited only to a small amount of fine filler. Therefore, the brush abrasion resistance on the prosthetic surface was at such a low level that it could not be used clinically, and thus the staining was used only in the rigid resin sandwich method (internal stain method).

However, the sandwich method had negative factors such as those below:
1. The stain shade nuance changed depending on the hue and thickness of the rigid resin covering the stain, and therefore,
2. the sandwich method only allowed skilled technicians to use it precisely, and
3. it could not be used for the final adjustment.

In many countries the law prohibits dental technicians who are the principal technicians in dental technique to see the patient directly and participate in treatment. Therefore, the prosthetic is produced strictly under the instructions of a dentist. At such time, in many cases the hue specifications, are conducted according to a hue guide, e.g. the VITA^{®} Shade Guide which is classified into 16 colors. However, this cannot reflect the natural tooth color of a patient accurately, and thus a small offset in color and aberration has resulted in many cases. Also, natural teeth have opaque bands and dots, topological hue variations as well as discoloration from external factors (such as smoking), and thus differences between individuals are great.

Since rigid resin in dentistry requires a high surface smoothness, it is the general practice to use a small as possible diameter particle filler." In the early days of the conventional product, although there were some dentists using PMMA spherical particle, they had a big problem with durability (abrasion resistance) due to severe wear in the PMMA area. Ultra fine silica of approximately 40 nm in mean particle diameter that is produced through spray thermal decomposition of silicone tetrachloride is generally used for conventional products in recent years. For example, US 5,009,597 proposes a shell or jacket material for crowns which contains 30-70% by weight of microdispersed silicon dioxide, preferably of a particle size from 0.01-0.04 micrometers. EP 475 239 B1 concerns a filler mixture of (A) nano-sized spherical SiO₂ with 20% high refractive index oxides and (B) micro-sized quartz, ceramic or glass powder, as well as optionally pyrogenic silica as rheological modifier. DE 196 157 63 A1 describes a filler of porous SiO₂ glass of 20-120 nm which is purported to give good abrasion resistance in dental composite materials. DE 44 46 033 C2 relates to fine particle silica fillers with a sheet crystal structure.

However, when such ultra fine particles are used, the completed resin paste increases the viscosity rapidly when the amount of ultra fine particle increases, and thus the resin paste becomes very difficult to handle in terms of dental technique operation. Therefore, the conventional product generally has a problem of not being able to increase the filler content higher than a certain amount.

In rigid resin for dental technique (for crown restoration), to some degree a solution has been found thanks to the development of organic composite filler. The organic composite filler was created by crushing the paste with a high content ratio of thoroughly kneaded ultra fine particle filler after it was cured. Since it was a filler with a mean particle diameter of several µm or more, the paste containing the filler shows characteristics and conditions as if it were a crushed type of filler containing resin. It behaves like an ultra fine filler containing resin when grinding it that makes it easy to obtain a very smooth surface. Nonetheless, from the viewpoint of the inorganic filler content's amount, it remains at approximately 50% by mass, and the mechanical characteristic was not all that high.

However, for the light curing type of paint resin for shade adjustment, it is desirable to have a paint resin for the purpose of hue adjustment of the rigid resin veneered crown. In other words, it is necessary to make a paste having sufficient viscosity to make an application work with use of a brush possible while improving the mechanical strength. In particular, wear and abrasion resistance capability is indispensable.

Conventional rigid resins have to be built up by use of such means as a spatula for shaping, the paste must be in clay form with a certain shape retaining capability. However, since the stain material for such purpose, as the light curing type of paint resin for shade adjustment in this patent application, requires a paint resin application and fine manipulation with use of a brush, it must be in a gel form paste.

DE 38 39 069 A1 describes a method for coating dental restorative parts produced by CAD/CAM methods. A thin pigmented coating is applied, followed by a second glossy anti-abrasive transparent sealant. The sealant has a preferred inorganic filler content of more than 40% and is preferably ultra fine particle silica. The pigmented coating should be of low viscosity to give a very thin layer. Abrasion resistance is not a requirement for the pigmented coating.

EP 1 243 230 A2 discloses a composition for prosthesis repair and orthodontic work comprising a matrix resin, e.g. urethane dimethacrylate, bis-GMA, triethylene glycol dimethacrylate, a filler mixture, e.g. pyrogenic SiO₂ / splinter polymerisates (SiO₂ /polydodecanediol dimethacrylate), polymerisation initiators, e.g. camphorquinone, and dental pigments..

US 4 411 625 discloses a dental restorative composition with improved colour stability comprising, a matrix resin, e.g. bis-GMA, triethylene glycol dimethacrylate, fillers, e.g. silanated silica, silanated barium aluminium silicate, etc. initiators, e.g. camphorquinone, and dental pigments, e.g. iron oxide pigments.

US 4 150 485 disc loses a low viscosity (less than or equal to 5 Pa.s) dental restorative coating composition comprising a matrix resin, e.g. bis-GMA, triethylene glycol dimethacrylate, fillers, Initiators (e.g. redox-type benzoyl peroxide/aromatic amine - cf. present application, description, page 5, line 21), and dental pigments. However, D3 does not disclose a light polymerisation catalyst nor does D3 disclose splinter Polymers.

The object of the present invention is to develop stain material for the final shade adjustment as a tool which can be used for a prosthetic surface (e.g. all kinds of crowns, bridges, veneers, inlays) and artificial teeth (e.g. removable or fixed, particial- and full dentures), enabling the user to produce a prosthetic with a hue of more natural appearance close to the natural dental color of a patient. Desired is a photocurable coating material for color correction of dental restorations or teeth, which is flowable, of paste-like or paint-like viscosity, applicable by a brush or similar instrument, capable to give a smooth and glossy surface after curing, and which produces an abrasion resistant coating on the surface of a tooth or a dental restoration in a desired color shade.

The invention accordingly relates to a method of adjusting the color of dental restorative parts by applying a dental coating material to the surface of the part and curing the layer, which coating material is a single paste composition comprising a photoinitiator and comprises
(A) a matrix resin:
(B) a filler mixture
(C) one or more polymerization initiator(s)
(D) trace quantities of one or more dental pigments
and which has a dynamic viscosity as measured on a plate-plate system from 1.0 x 10⁴ to 4 x 10⁴ [mPas] (average measured at 23°C and a shear rate of 10 sec⁻¹) and 10 x 10⁴ to 160 x 10⁴ [mPas] (average at 0.1 sec⁻¹, measured at 23°C 40 sec after shear rate reduction);
wherein the filler mixture (B) comprises fillers from the group of silicon dioxide, dental glass (e.g Ba-Al-Silica-glass), further metal- and non-metal oxides or their mixed oxides, and surface treated silicon dioxide splinter polymer.

In one embodiment the object of the present invention is achieved by a material comprising a matrix resin and an inorganic filler - while maintaining sufficient fluidity for making an application of the paint resin with a brush possible. The material comprises (A) 40 - 60% by mass of matrix resin, (B) 60 - 40% by mass of a filler mixture; (C) 0.1 - 1% by mass of one or more polymerization initiator(s); and (D) trace quantities of one or more dental pigments.

When implementing the invention, an existing widely known matrix resin can be used (Shin Zairyo Shin Sozai Series Saishin Shika Zairyo Oyobi Gijutsu Kiki (New Materials Series: Latest Dental Materials), edited by Hasegawa Jiro (Jiro Hasegawa) and published by Kabushikigaisha CMC, Pages 7 18). Normally, methacrylate monomer, especially polymerization multifunctional methacrylate, which can form a bridge structure after curing, is used as the matrix monomer by considering safety for the organism.

Suitable free radically-polymerizable monomers may contain at least one ethylenically-unsaturated bond, can be oligomers or polymers, and are capable of undergoing addition polymerization. Such monomers include mono-, di- or poly- acrylates and methacrylates such as methyl acrylate, methyl methacrylate, ethyl acrylate, isopropyl methacrylate, n-hexyl acrylate, stearyl acrylate, allyl acrylate, glycerol diacrylate, glycerol triacrylate, ethyleneglycol diacrylate, diethyleneglycol diacrylate, triethyleneglycol dimethacrylate, 1,3-propanediol diacrylate, 1,3-propanediol dimethacrylate, trimethylolpropane triacrylate, 1,2,4-butanetriol trimethacrylate, 1-4-cyclohexanedio diacryl ate, pentaerythritol triacrylate, pentaerythritol tetraacrylate, pentaerythritol tetramethacrylate, sorbitol hexacrylate, bis[1-(2-acryloxy)]-p-ethoxyphenyldimethylmethane, bis[1-(3-acryloxy-2-hydroxy)]-p-propoxyphenyldimethylmethane, tris(hydroxyethylisocyanurate)-trimethacrylate; the bis-acrylates and bis-methacrylates of polyethylene glycols of molecular weight 200-500, copolymerizable mixtures of acrylated monomers such as those of U.S. Pat. No. 4,652,274, incorporated herein by reference, and acrylated oligomers such as those of U.S. Pat. No. 4,642,126, incorporated herein by reference; unsaturated amides such as methylene bisacrylamide, methylene bis-methaaylamide, 1,6-hexamethylene bisacrylamide, diethylene triamine tris-acrylamide and beta-methacrylamino-ethyl methacrylate; and vinyl compounds such as styrene, diallyl phthalate, divinyl succinate, divinyl adipate and divinylphthalate. Mixtures of two or more monomers can be used if desired. Preferably, the free radically polymerizable material used is mono-, di-, or poly-acrylates and methacrylates such as methyl acrylate, methyl methacryle, ethyl acrylate, glycidyl methacrylate, 2-isocyanatoethyl methacrylate, limonene oxide, isopropyl methacrylate, n-hexyl acrylate, stearyl acrylate, allyl acrylate, glycerol diacrylate, glycerol triacrylate, ethyleneglycol diacrylate, diethyleneglycol diacrylate, triethyleneglycol dimethacrylate, 1,3-propanediol diacrylate, 1,3-propanediol dimethacrylate, trimethylolpropane triacrylate, 1,2,4-butanetriol trimethacrylate, 1,4-cyclhexanediol diacrylate, penterythritol triacrylate, pentaerythritol tetracrylate, pentaerythritol tetramethacrylate, sorbitol hexacrylate, bis[1-929acryloxy)]-p-ethosyphenyl dimethylmethane, bis[1-(3-acryloxy-2-hydroxy)}-p-propoxy-phenyldimethylmethane, and trihydroxyethyliso-cyanurate trimethacrylate; the bisacrylates and bis-methacryles of polyethylene glycols of molecular weight 200-500, copolymerizable mixtures of acrylated monomers such as those in U.S. Pat. No. 4,652,274, and acrylated oligomers such as those of U.S. Pat. No. 4,642,126; and vinyl compounds such as styrene, diallyl phthalate, divinyl succinate, divinyl adipate and divinylphthalate. Mixtures of two or more of these free radically polymerizable materials can be used if desired.

Two types of matrix monomers are mainly used, and they are: bisphenol A, having a relatively large molecular weight and small shrinkage after cure, as the fundamental structure and another one having a urethane structure. It is common practice to combine them with monomers such as a dimethacrylate monomer having an ethylene glycol chain for the purpose of adjusting viscosity and, refraction index for use.

Preferred monomers are bis-GMA, Bisphenol-A-Ethoxydimethacrylate , 2,2-bis[4-(2-hydroxy-3-methacryloxypropoxy)phenyl]propane, polymeric ethoxylated Bisphenol A dimethacrylates (Bis-EMA), Bis EMA (2,6), Bis EMA(6), triethylene glycol dimethacrylate (TEGDMA), 1,6-bis(methacsyloxy-2-ethoxycarbonylamlno)-2,4,4-trimethylhexan (UDMA).

A widely known existing initiator (light polymerization catalysis) can be used (New Materials Series: Latest Dental Materials), edited by Hasegawa Jiro (Jiro Hasegawa) and published by Kabushikigaisha CMC, Page 19). Radical polymerization is normally employed for a composite resin curing reaction. The initiators are classified into the chemical polymerization type products that use a redox initiator composed of a combination of benzoyl peroxide/aromatic amine (R3N), and the light curing type of products that use a visible light polymerization initiator composed of a combination of camphorquinone/aromatic amine (R3N). However, single pastes of them are possible, and thus the mainstream is the light curing type products with no grinding required and no air bubble contamination. Examples of suitable photoinitiators are benzophenone, benzoin and their derivatives or alpha-diketones and their derivatives, such as 9,10-phenanthrenequinone, diacetyl or 4,4-dichlorobenzil; and alpha-diketones in combination with amines as reducing agents, such as e.g. cyanoethylmethylaniline, mathylaminoethylmethacrylate, triethanolamine, N,N-dimethyl-sym.-xylidine. Further suitable photoinitiators are acyl phosphines, such as e.g. 2,4,6-trimethylbenzoyl-dipherlyl- or bis(2,6-dichlorobenzoyl)-4-N-propylphenylphosphinoxide.

Camphorquinone is especially suitable.

A widely known existing filler mixture, which is made of silicon dioxide and surface treated silicon dioxide, can be used (New Materials Series: Latest Dental Materials), edited by Hasegawa Jiro (Jiro Hasegawa) and published by Kabushikigaisha CMC, Pages 16 17). A typical composite resin can be classified by the type of a large amount of the filler used for filling it. For example, it can be classified by particle diameter such as follows:
(1) Larger than 1 µm (macro particle filling type)
(2) 0.1 µm -1 µm (sub micron particle filling type)
(3) Smaller than 0.1 µm (ultra fine particle filling type)
(4) A mixture of different diameter particle fillers of the above.

In some cases, it is classified by a diameter of 3 µm. As for particle geometry, there are shapeless, spherical shape and fabric shape.
When viewed from the composition aspect, barium aluminosilicate glass, silica, zirconia, metal and non metal oxides and their mixtures and cracked polymer (splinter poylmer) containing ultra fine particles can be listed. Although various treatment agents as well as methods are employed for such surface treatment, normally a polymerization functional group is introduced to the silanol on the filler surface by a silane compound such as a gamma-methacryl oxypropyl trimethoxy silane (Silane A 174) in order to gain affinity to the matrix.

The filler mixture of the present invention is preferably made up of silicon dioxide 40 - 60 % by mass and silicone dioxide splinter polymer 60 - 40 % by mass. The silicon dioxide splinter polymer is preferably silicon dioxide/polydodecanediol dimethacrylate. Of course similar splinter polymers will also be suitable for the compositions of the invention. Suitable splinter polymers are produced by polymerizing (meth)acrylate monomers in the presence of silicon dioxide powders an subsequent grinding of the resulting material.

A preferred composition in the method according to the invention comprises matrix resin about 50% by mass, filler mixture about 49% by mass, and initiator about 1% by mass.

Especially preferred are compositions wherein: bisphenol A diglycidyl acrylate 19 % by mass, urethane methacrylate 11% by mass, triethylene glycol dimethacrylate 22% by mass, silicon dioxide 23% by mass, silicon dioxide/polydodecanediol dimethacrylate 24,4 % by mass, and initiator 0.6% by mass.

The intended use for shade adjustment purposes usually requires the presence of pigments, that are common in the dental field.

Especially preferred pigments are iron-oxides, chromium-iron-zino-spinelles, titanium-dioxides, copper-chromium-iron-spinelles, cobalt-alumina-spinelles and zirconium oxides.

Pastes especially suitable for the described usage have preferably the following viscosic behaviour:

### Viscosity test:

Equipment: Universal Dynamic Rheometer UDS 200 (dynamic viscosity. measuring device)
Measuring apparatus:
   Plate plate system; upper plate MP30, 25 mm diameter, 0°, sand blasted; lower plate sand blasted.
Conditions: temperature when measuring was conducted: 23°C; gap width 0.5 mm; waiting time before measurement starts: 3 min.
Description: Verify that material is filled evenly in the gap and let the upper plate rotate to initiate measurement. Measure the average viscosity over 180 sec at a shear rate of 10 sec⁻¹ . Reduce the shear rate to 0.1 sec⁻¹ . Collect the recovery of the viscosity 40 sec after the reduction of the shear rate. Repeat measurements several times with freshly applied material.
   The results are shown in Table 1.

**Table 1**

| Material | Value |
|---|---|
| Average viscosity at a shear rate of 10 sec⁻¹ | 1.0 x 10⁴ to 4 x 10⁴ [mPas] |
| Average viscosity at 0.1 sec⁻¹ measured 40 sec after shear rate reduction (viscosity recoverage) | 10 x 10⁴ to 160 x 10⁴ [mPas] |

Pastes especially suitable for the described usage have the following abrasive behaviour: Tooth brush wear and abrasion test. Comparison of physical properties with a conventional material

| | |
|---|---|
| Medium: Odolmed (tooth paste): Water = 2 : 1 | Cycles: 100, 000 |
| Load: 200 grams | Motion: Slow |

### Material in this test:

Bisphenol A diglycidyl acrylate 19 % by mass, urethane methacrylate 11% by mass, triethylene glycol dimethacrylate 22% by mass, silicon dioxide 23% by mass, silicon dioxide/polydodecanediol dimethacrylate 24,4 % by mass, and initiator 0.6% by mass.

### Dentacolor Sirius (registered trademark); composition:

Matrix resin (urethane dimethacrylate, triethylene glycol dimethacrylate, dodecane diol methacrylate) 25.5% by mass; filler (silicon dioxide/polydodecanedioldimethacrylate, silicon dioxide) 74 % by mass; catalyst (camphorquinone) 0.5 % by mass; and trace quantity of pigment.

Comparison of physical properties data of prefered material and Dentacolor Sirius are shown in Table 2

**Table 2:**

| | Wear amount | Wear depth | Filling ration[%] | Surface roughness |
|---|---|---|---|---|
| Preferred material | 0.1040 mm³ | 6.6 µm | 47,4 | 0.41 µm |
| Dentacolor Sirius | 0.1985 mm³ | 13.8 µm | 78 | 0.85 µm |

## Claims

1. A method of adjusting the color of dental restorative parts by applying a dental coating material to the surface of the part and curing the layer, which coating material is a single paste composition comprising a photoinitiator and comprises
(A) a matrix resin:
(B) a filler mixture
(C) one or more polymerization initiator(s)
(D) trace quantities of one or more dental pigments
and which has a dynamic viscosity as measured on a plate-plate system from 1.0 × 10⁴ to 4 x 10⁴ [mPas] (average measured at 23°C and a shear rate of 10 sec⁻¹) and 10 x 10⁴ to 160 x 10⁴ [mPas] (average at 0.1 sec⁻¹, measured at 23°C 40 sec after shear rate reduction);
wherein the filler mixture (B) comprises fillers from the group of silicon dioxide, dental glass (e.g Ba-Al-Silica-glass), further metal- and non-metal oxides or their mixed oxides, and surface treated silicon dioxide splinter polymer.

2. A method according to claim 1, wherein the dental coating material comprises
(A); 40 - 60% by mass
(B): 60 - 40% by mass
(C): 0.1 - 1% by mass

3. A method according to claim 1 or 2, wherein the filler mixture (B) comprises 40 or more wt.% of surface treated silicon dioxide and 60 or less wt.% of surface treated silicon dioxides splinter polymer.

4. A method according to any of claims 1 to 3, in which the splinter polymer is surface treated silicon dioxide/polydodecane diol dimethacrylate.

5. A method according to any of claims 1 to 4, wherein the fillers are completely or partly surface treated.

6. A method according to any of claims 1 to 5 wherein the matrix resin (A) is a mixture of bisphenol A diglycidyl acrylate, urethane dimethacrylate and triethylene glycol dimeth-acrylate.

7. A method according to any of claims 1 to 6 comprising matrix resin (A) about 50% by mass, filler mixture (B) about 49% by mass and initiator (C) about 1% by mass.

8. A method according to any of claims 1 to 6, wherein the main initiator is camphorquinone.

9. A method according to any of claims 1 to 8 wherein the dental coating material comprises bisphenol A diglycidyl acrylate, about 20% by mass; urethane dimethacrylate about 10% by mass; triethylene glycol dimethacrylate about 20% by mass, silicon dioxide about 20% by mass; silicon dioxide/ polydodecanediol dimethacrylate about 20% by mass and initiator about 0.6% by mass.

## Patentansprüche

1. Verfahren zur Anpassung der Farbe von Zahnrestaurationsteilen durch Auftragung eines Zahnbeschichtungswerkstoffs auf die Oberfläche des Teils und Aushärtung der Schicht, wobei der Beschichtungswerkstoff eine einzelne Pastenzusammensetzung ist, umfassend einen Photoinitiator und umfassend
(A) ein Matrixharz
(B) eine Füllstoffmischung,
(C) ein oder mehrere Polymerisationsinitiator(en)
(D) Spurenmengen eines oder mehrerer Zahnpigmente,
und eine dynamische Viskosität, bei Messung mit einem Platte/Platte-System, von 1,0 x 10⁴ bis 4 x 10⁴ [mPas] (Durchschnitt gemessen bei 23°C und einer Scheerrate von 10 sec⁻¹) und von 10 x 10⁴ bis 160 x 10⁴ [mPas] (Durchschnitt bei 0,1 sec⁻¹, gemessen bei 23°C 40 Sek. nach Senkung der Scheerrate) besitzt,
wobei die Füllstoffmischung (B) Füllstoffe aus der Gruppe Siliziumdioxid, Dentalglas (z.B. Ba-AI-Silika-Glas), weitere Metall- und Nichtmetalloxide oder deren Mischoxide und oberflächenbehandelten Siliziumdioxid-Splitterpolymer umfasst.

2. Verfahren nach Anspruch 1, bei dem der Zahnbeschichtungswerkstoff umfasst
(A): 40-60 Masse-%
(B): 60-40 Masse-%
(C): 0,1-1 Masse-%.

3. Verfahren nach Anspruch 1 oder 2, bei dem die Füllstoffmischung (B) 40 oder mehr Gew.% oberflächenbehandeltes Siliziumdioxid und 60 oder weniger Gew.% oberflächenbehandelten Siliziumdioxid-Splitterpolymer umfasst.

4. Verfahren nach einem der Ansprüche 1 bis 3, bei dem es sich bei dem Splitterpolymer um oberflächenbehandeltes Siliziumdioxid/Polydodekandioldimethacrylat handelt.

5. Verfahren nach einem der Ansprüche 1 bis 4, bei dem die Füllstoffe vollständig oder teilweise oberflächenbehandelt sind.

6. Verfahren nach einem der Ansprüche 1 bis 5, bei dem es sich bei dem Matrixharz (A) um eine Mischung aus Bisphenol A-Diglycidylacrylat, Urethandimethacrylat und Triethylenglykoldimethacrylat handelt.

7. Verfahren nach einem der Ansprüche 1 bis 6, umfassend ungefähr 50 Masse-% Matrixharz (A), ungefähr 49 Masse-% Füllstoffmischung (B) und ungefähr 1 Masse-% Initiator (C).

8. Verfahren nach einem der Ansprüche 1 bis 6, bei dem es sich bei dem Hauptinitiator um Kampferchinon handelt.

9. Verfahren nach einem der Ansprüche 1 bis 8, bei dem der Zahnbeschichtungswerkstoff ungefähr 20 Masse-% Bisphenol A-Diglycidylmethacrylat, ungefähr 10 Masse-% Urethandimethacrylat, ungefähr 20 Masse-% Triethylenglykoldimethacrylat, ungefähr 20 Masse-% Siliziumdioxid, ungefähr 20 Masse-% Siliziumdioxid/Polydodekandioldimethacrylat und ungefähr 0,6 Masse-% Initiator umfasst.

## Revendications

1. Procédé d'ajustement de la couleur de parties de restauration dentaire par application d'un matériau de revêtement dentaire sur la surface de la partie et en durcissant la couche, dont le matériau de revêtement est une composition de pâte unique comprenant un amorceur photochimique et qui comprend :
(A) une résine matrice,
(B) un mélange de charges,
(C) un ou plusieurs amorceurs de polymérisation,
(D)des quantités à l'état de traces d'un ou de plusieurs pigments dentaires.
et qui a une viscosité dynamique, mesurée sur un système plateau sur plateau, de 1,0 x 10⁴ à 4 x 10⁴ [mPas] (moyenne mesurée à 23°C avec un taux de cisaillement de 10 sec⁻¹) et de 10 x 10⁴ à 160 x 10⁴ [mPas] (moyenne pour 0,1 sec⁻¹, mesurée à 23°C, 40 secondes après réduction du taux de cisaillement);
dans laquelle le mélange de charges (B) comprend des charges constituées de dioxyde de silicium, de verre dentaire (par exemple verre Ba-Al-silice), en plus des oxydes métalliques et non métalliques ou leurs oxydes mélangés et des polymères en éclats avec du dioxyde de silicium traité en surface.

2. Procédé selon la revendication 1, **caractérisé en ce que** le matériau de revêtement dentaire comprend :
(A) : 40 à 60 % en masse,
(B) : 60 à 40 % en masse,
(C) : 0,1 à 1 % en masse,

3. Procédé selon la revendication 1 ou 2, **caractérisé en ce que** le mélange de charges (B) comprend 40 % ou plus en poids de dioxyde de silicium traité en surface et 60 % en poids ou moins de polymère en éclats avec du dioxyde de silicium traité en surface.

4. Procédé selon l'une quelconque des revendications 1 à 3, dans lequel le polymère en éclats est du dioxyde de silicium traité en surface/diméthacrylate de polydodécane diol.

5. Procédé selon l'une quelconque des revendications 1 à 4, **caractérisé en ce que** les charges sont complètement ou partiellement traitées en surface.

6. Procédé selon l'une quelconque des revendications 1 à 5, **caractérisé en ce que** la résine matrice (A) est un mélange d'acrylate de diglycidyl bisphénol A, d'un diméthacrylate uréthane, et de diméthacrylate de triéthylène glycol.

7. Procédé selon l'une quelconque des revendications 1 à 6, comprenant environ 50 % en masse de résine matrice (A), environ 49 % en masse du mélange de charges (B) et environ 1 % en masse de catalyseur (C).

8. Procédé selon l'une quelconque des revendications 1 à 6, **caractérisé en ce que** l'amorceur principal est la camphorquinone.

9. Procédé selon l'une quelconque des revendications 1 à 8, **caractérisé en ce que** le matériau de revêtement dentaire comprend de l'acrylate de diglycidyl bisphénol A, environ 20 % en masse ; un diméthacrylate uréthane, environ 10 % en masse ; du diméthacrylate de triéthylène glycol, environ 20 % en masse, du dioxyde de silicium, environ 20 % en masse ; du dioxyde de silicium/diméthacrylate de polydodécane diol, environ 20 % en masse et un amorceur, environ 0,6 % en masse.
